# FASCICULE DE BREVET EUROPEEN

(11) **EP 1 973 504 B1**
(45) Date de publication et mention de la délivrance du brevet: **09.12.2009**
(21) Numéro de dépôt: 07718341.6
(22) Date de dépôt: 19.01.2007
(51) Int. Cl.: A61F 5/02

(54) **CEINTURE DE SOUTIEN LOMBAIRE**
LUMBALER STÜTZGURT
LUMBAR SUPPORT BELT

(30) Priorité: 19.01.2006 FR 0600493
(43) Date de publication de la demande: 01.10.2008
(73) Titulaire: Gibaud, 42000 Saint Etienne (FR)
(72) Inventeur: ANQUETIL, Lionel, F-03110 Serbannes (FR)
(74) Mandataire: Delorme, Nicolas
(86) Numéro de dépôt international: PCT/FR2007/000108
(87) Numéro de publication internationale: WO 2007/083035

(56) Documents cités:
- EP-A- 1 062 925
- DE-A1- 2 334 500
- US-A- 3 970 079
- US-A- 5 226 874
- US-A- 5 626 616

## Description

La présente invention concerne une ceinture de soutien lombaire.

Une ceinture de soutien lombaire est une ceinture orthopédique dont le port peut être prescrit pour traiter un patient souffrant de "mal de dos".

Le mal de dos recouvre diverses pathologies telles que lombalgie, sciatique ou scoliose chroniques ou aigus.

Le traitement orthopédique de ce type de pathologie a deux objectifs. Il s'agit, d'une part, de diminuer la douleur ressentie par le patient, et d'autre part, de maintenir le patient dans une bonne position.

Ce traitement orthopédique peut passer par le port d'une ceinture lombaire qui est une ceinture en matière textile élastique renforcée par un baleinage postérieur et, éventuellement, antéro latéral.

La ceinture lombaire vient entourer la partie inférieure du tronc du patient et assure un maintien et une contention de la région lombaire ainsi que de la région abdominale du patient.

Grâce au textile élastique dans lequel elle est coupée et grâce au baleinage dont elle est pourvue, la ceinture lombaire assure un rapport de posture qui s'avère bénéfique pour le patient.

Le port d'une ceinture de soutien lombaire peut également être prescrit, à titre préventif, pour éviter au patient de reproduire des gestes néfastes.

Il apparaît donc que le port d'une ceinture de soutien lombaire peut être bénéfique pour un large nombre de patients.

On connaît par le document US-A-5 226 874 une ceinture de toile dont l'élasticité est conférée par des bandes élastiques et qui possède des renforts rigides qui sont peu confortables et ne favorisent pas l'observance.

Le document EP-A-1062525 montre un système à base de cales qui prennent appui sur une surface telle qu'un matelas pour appliquer une pression sur le patient. Ce système ne fonctionne donc que lorsque le patient est couché ou assis sur une surface d'appui.

Le document US-A-3 970 079 montre une ceinture thoracique constituée de panneaux rectangulaires et trapézoïdaux. Ces panneaux sont reliés entre eux par des éléments de PVC extrudés. Ces éléments de PVC extrudés présentent une rigidité qui rend la ceinture inconfortable.

Le port d'une ceinture de soutien lombaire peut donc s'avérer inconfortable.

Notamment en position assise, le patient peut ressentir une compression abdominale au niveau de l'estomac.

L'inconfort, essentiellement en position assise, induit par le port d'une ceinture de soutien lombaire, ne favorise pas l'observance du traitement et certains patients, dont l'activité se déroule principalement en position assise, peuvent être tentés de cesser de porter la ceinture de soutien lombaire qui leur a été prescrite.

Un but de l'invention est de proposer une ceinture de soutien lombaire qui tout en réalisant un rappel de posture efficace soit d'un port confortable.

L'invention a pour objet une ceinture de soutien lombaire, qui peut être mise en place sur la partie inférieure du tronc d'un patinent ; elle comprend une bande de textile élastique dotée à chacune de ses extrémités de moyens d'accrochage de la bande sur elle-même et au moins deux éléments de baleinage postérieures en matière élastique disposés de part et d'autre du plan transversal médian de la bande ; cette ceinture de soutien lombaire peut être positionnée sur le patient de telle sorte les extrémités de la bande s'accrochent en regard de la zone abdominale du patient et les éléments de baleinage postérieurs viennent en appui contre la zone lombaire du patient ; en outre, les éléments de baleinage postérieurs sont orientés selon un V de telle sorte que les éléments de baleinage postérieurs convergent vers la région lombo-sacrée du patient.

Par la disposition en V de ses éléments de baleinage, la ceinture de soutien lombaire selon l'invention présente un double effet. D'une part, elle assure un rappel de posture très efficace et, grâce à la convergence des baleines, assure une concentration de l'appui élastique dans la zone lombosacrée qui est bien souvent le siège des douleurs ressenties par le patient. D'autre part, la ceinture de soutien lombaire selon l'invention s'avère d'un port sensiblement plus agréable que celui d'une ceinture classique dans la mesure où les éléments de baleinage sont orientés de manière divergente en direction des côtes flottantes du patient. Cette disposition fait que, notamment, lorsque le patient est en position assise, l'écartement de l'extrémité supérieure des éléments de baleinage limite la compression sur l'estomac du patient.

Il est, par ailleurs, envisagé que la bande de textile élastique soit pourvue d'au moins deux pinces transversales et symétriques par rapport au plan médian.

La présence de pinces produit un creusement de la ceinture de soutien lombaire selon l'invention qui lui donne une forme de sablier lorsqu'elle est portée. La présence de ces pinces combinées à la disposition en V des éléments de baleinage contribue à encore améliorer le confort du patient en limitant la compression de l'estomac.

Selon une forme d'exécution, chaque élément de baleinage postérieur présente deux baleines juxtaposées parallèles.

De plus, la ceinture de soutien lombaire peut présenter deux baleines antérolatérales parallèles entre elles et disposées symétriquement par rapport au plan transversal médian.

Pour accentuer l'effet de creusement de la ceinture de soutien lombaire, il peut être envisagé que celle-ci présente une pince sensiblement superposée à chacune des baleines antérolatérales.

La ceinture de soutien lombaire peut en outre présenter deux baleines antérieures parallèles entre elles et disposées symétriquement par rapport au plan transversal médian.

Dans une forme d'exécution, la bande de textile élastique présente une partie centrale qui reçoit les éléments de baleinage disposés en V prolongée par deux parties latérales qui reçoivent chacune une baleine antérolatérale et une baleine antérieure, la partie centrale susceptible de venir en appui contre la zone lombaire du patient étant élargie par rapport à chacune des parties latérales.

On peut envisager, de façon pratique, que les extrémités de la ceinture de soutien lombaire soient pourvues de tissu à boucles et crochets permettant de fermer celle-ci sur elle-même.

Pour sa bonne compréhension, l'invention est décrite en référence au dessin ci annexé représentant, à titre d'exemple non limitatif, une forme de réalisation d'une ceinture de soutien lombaire selon celle-ci.
Figure 1 montre une ceinture de soutien lombaire selon l'invention mise à plat,
Figures 2 et 3 montrent cette ceinture de soutien lombaire placée sur un patient, respectivement en vue postérieure et en vue latérale.

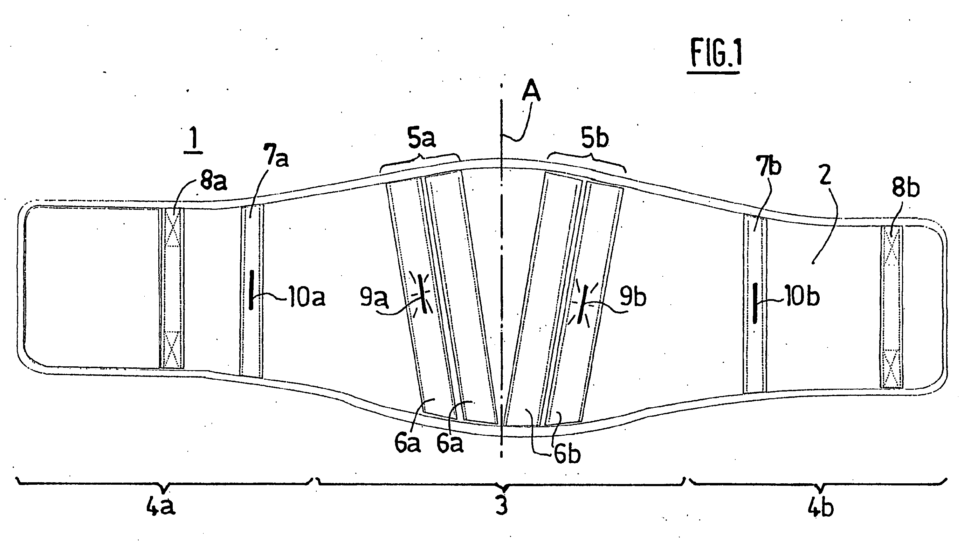

En se reportant tout d'abord à la figure 1, il apparaît que la ceinture de soutien lombaire 1 dans sa forme de réalisation illustrée présente une bande de matière textile. La ceinture de soutien lombaire est réalisée dans un textile élastique à base notamment de fils d'élasthanne. L'élasticité est présente notamment dans le sens longitudinal de la bande 2.

On peut noter que la ceinture de soutien lombaire, dans sa forme de réalisation illustrée sur le dessin, présente une partie centrale 3 prolongée par deux parties latérales symétriques 4a et 4b.

La partie centrale 3 est élargie par rapport à chacune des parties latérales 4a et 4b. De façon tout à fait spécifique à la ceinture de soutien lombaire selon l'invention, celle-ci est pourvue de deux éléments de baleinage postérieurs 5a, 5b qui sont disposés de manière convergente par rapport à l'axe médian transversal A.

De façon tout à fait caractéristique, les deux éléments de baleinage postérieurs sont disposés selon un V de part et d'autre de l'axe médian transversal A.

Dans la forme d'exécution de la ceinture de soutien lombaire représentée sur le dessin, chaque élément de baleinage postérieur est constitué de deux baleines parallèles 6a, 6b. Les baleines 6a, 6b sont des éléments plats réalisés dans une matière élastique de type acier ressort ou matériau composite et sont engagés dans des goussets qui, dans l'exemple représenté, sont cousus sur la bande de textile élastique 2.

Chacune des parties latérales 4a, 4b est, quant à elle, pourvue de deux baleines, à savoir une baleine antérolatérale 7a, 7b et une baleine antérieure 8a, 8b. Contrairement aux éléments de baleinage postérieurs qui, comme on l'a vu, sont disposés en V, les baleines 7a, 7b, 8a, 8b de chacune des parties latérales sont parallèles à l'axe médian transversal.

De manière également tout à fait caractéristique à la ceinture de soutien lombaire suivant l'invention, celle-ci présente plusieurs pinces 9, 10. Ces pinces 9a, 9b, 10a, 10b peuvent être au nombre de quatre et se répartirent en pinces postérieures 9a, 9b qui sont superposées aux éléments de baleinage postérieur 5a, 5b et en pinces 10a, 10b antérolatérales qui se superposent à chacune des baleines antérolatérales.

On verra plus loin l'effet combiné de ces pinces et des éléments de baleinage disposés en V. Chacune de ces pinces est réalisée de manière classique par la découpe d'un élément de matière dans la bande élastique 2, les bords de la zone ainsi découpée sont ensuite rapprochés et cousus entre eux.

Il est à remarquer que les extrémités de la ceinture de soutien lombaire sont respectivement munies de bandes de tissu à boucles et crochets.

La mise en place de la ceinture de soutien lombaire selon l'invention sur un patient se fait de la manière suivante.

La partie centrale 3 de la ceinture de soutien lombaire vient se plaquer contre la zone lombaire du patient tandis que les deux parties latérales 3a, 3b de la ceinture de soutien lombaire viennent se rencontrer et s'accrocher l'une à l'autre au niveau de la zone abdominale du patient.

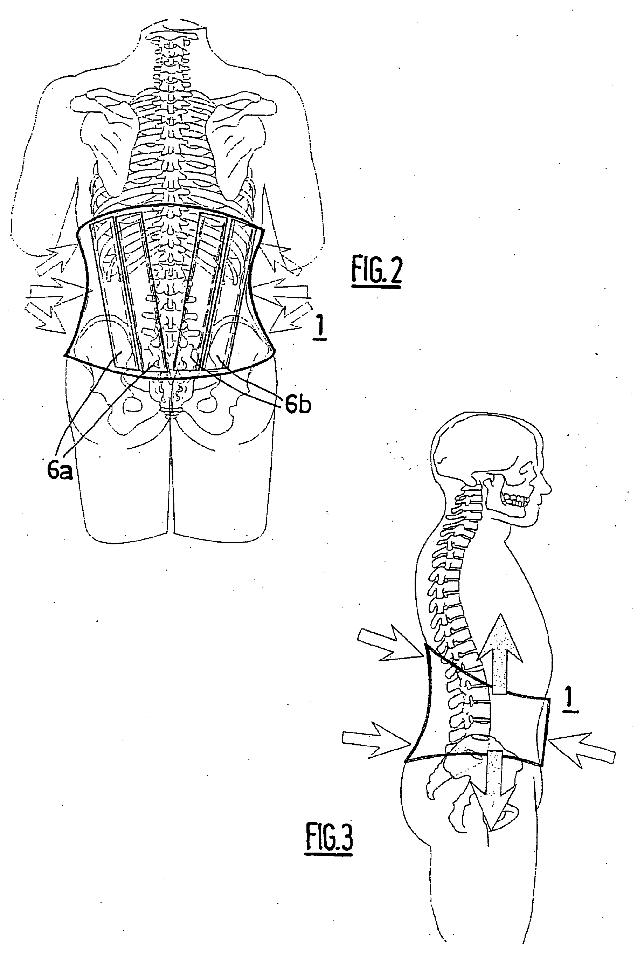

La figure 2 illustre tout à fait clairement les deux points essentiels de la ceinture de soutien lombaire selon l'invention, à savoir la disposition en V des éléments de baleinage qui convergent vers la charnière lombo sacrée du patient et la disposition resserrée en sablier de la ceinture de soutien lombaire lorsqu'elle est mise en place sur un patient.

Un premier effet tout à fait significatif de la disposition en V des éléments de baleinage postérieur 5a, 5b est une concentration de l'élasticité produite par les éléments de baleinage dans la région lombosacrée qui est généralement le siège de douleurs en cas de lombalgie ou de sciatique.

Un second effet de la disposition en V des éléments de baleinage est que, lorsque le patient est dans une position assise, le fait que les éléments de baleinage soient divergents au niveau des cotes flottantes fait que la compression sur l'estomac et la gêne qui peut en résulter, sont considérablement limitées par rapport à une ceinture de soutien lombaire classique dans laquelle les éléments de baleinage sont parallèles entre eux.

Le confort, qui en résulte, favorise bien entendu l'observance de port de cette ceinture de soutien lombaire.

Ce deuxième effet, qui participe au confort du port, est favorisé par le creusement de la taille qui découle de la présence des pinces dans la ceinture.

Les flèches des figures 2 et 3 montrent la contention que réalise la ceinture de soutien lombaire selon l'invention et illustre l'élongation qui produit un déchargement du poids du corps sur les vertèbres basses.

La ceinture de soutien lombaire selon l'invention offre donc un effet orthopédique tout à fait remarquable puisqu'elle concentre son effet de rappel de posture au niveau de la charnière lombo sacrée et, corollairement, offre un confort d'utilisation très supérieur à celui des ceintures de soutien lombaires connues, puisqu'elle évite le phénomène de compression notamment en position assise.

Bien entendu, l'invention n'est pas limitée à la forme de réalisation décrite ci-dessus à titre d'exemple non limitatif, mais elle en embrasse au contraire toutes les formes de réalisation.

## Revendications

1. Ceinture de soutien lombaire (1), pouvant être mise en place sur la partie inférieure du tronc d'un patient, comprenant une bande (2) de textile élastique dotée à chacune de ses extrémités de moyens d'accrochage de la bande sur elle-même et au moins deux éléments de baleinage postérieures (5a, 5b) disposés de part et d'autre du plan (A) transversal médian de la bande, la dite ceinture pouvant être positionnée sur le patient de telle sorte les extrémités de la bande s'accrochent en regard de la zone abdominale du patient et les éléments de baleinage postérieurs (5a, 5b) viennent en appui contre la zone lombaire du patient, les éléments de baleinage postérieurs (5a, 5b) étant orientés selon un V de telle sorte que les éléments de baleinage postérieurs (5a, 5b) convergent vers la région lombo-sacrée du patient **charactérisée en ce que** les éléments de baleinage postérieures sont en matière élastique.

2. Ceinture de soutien lombaire (1) selon la revendication 1 **caractérisée en ce que** la bande(2) de textile élastique est pourvue d'au moins deux pinces (9a, 9b) transversales et symétriques par rapport au plan médian.

3. Ceinture de soutien lombaire (1) selon la revendication 1 ou ta revendication 2, **caractérisée en ce que** chaque élément de baleinage postérieur (5a, 5b) présente deux baleines (6a, 6b) juxtaposées parallèles.

4. Ceinture de soutien lombaire (1) selon l'une des revendications 1 à 3, **caractérisée en ce qu'**elle présente deux baleines antérolatérales (7a, 7b) parallèles entre elles et disposées symétriquement par rapport au plan transversal médian (A).

5. Ceinture de soutien lombaire (1) selon la revendication 4, **caractérisée en ce qu'**elle présente une pince (10a, 10b) sensiblement superposée à chacune des baleines antérolatérales (7a, 7b).

6. Ceinture de soutien lombaire (1) selon l'une des revendications 1 à 4, **caractérisée en ce qu'**elle présente deux baleines antérieures (8a, 8b) parallèles entre elles et disposées symétriquement par rapport au plan transversal médian (A).

7. Ceinture de soutien lombaire (1) selon l'une des revendications 1 à 6, **caractérisé en ce que** la bande de textile élastique présente une partie centrale (3) qui reçoit les éléments de baleinage postérieurs (5a, 5b) disposés en V, prolongée par deux parties latérales (4a, 4b) qui recoivent chacune une baleiné antérolatérale (7a, 7b) et une baleine antérieure (8a, 8b), la partie centrale (3) susceptible de venir en appui contre la zone lombaire du patient étant élargie par rapport à chacune des parties latérales (4a, 4b).

8. Ceinture de soutien lombaire selon l'une des revendications 1 à 7, **caractérisée en ce que** les extrémités de la ceinture sont pourvues de tissu à boucles et crochets permettant de fermer la ceinture sur elle-même.

## Claims

1. A lumbar support belt (1) which can be fitted around the lower portion of a patient's trunk, comprising a strip (2) of elastic textile material provided at each end with means for attaching the strip to itself and at least two posterior boning elements (5a, 5b) positioned on each side of the transverse mid-plane (A) of the strip, said belt being able to be positioned on the patient such that the ends of the strip fasten together over the abdominal region of the patient and the posterior boning elements (5a, 5b) press against the lumbar region of the patient, the posterior boning elements (5a, 5b) being arranged in a V-shape such that the posterior boning elements (5a, 5b) converge towards the lumbo-sacral region of the patient, **characterized in that** the posterior boning elements are made of elastic material.

2. The lumbar support belt (1) according to claim 1, **characterized in that** the strip (2) of elastic textile material is provided with at least two transverse clips (9a, 9b) symmetrical in relation to the mid-plane.

3. The lumbar support belt (1) according to claim 1 or claim 2, **characterized in that** each posterior boning element (5a, 5b) has two juxtaposed parallel bones (6a, 6b).

4. The lumbar support belt (1) according to one of claims 1 to 3, **characterized in that** it has two anterolateral bones (7a, 7b) parallel to each other and disposed symmetrically in relation to the transverse mid-plane (A).

5. The lumbar support belt (1) according to claim 4, **characterized in that** it has a clip (10a, 10b) essentially superimposed on each of the anterolateral bones (7a, 7b).

6. The lumbar support belt (1) according to one of claims 1 to 4, **characterized in that** it has two anterior clips (8a, 8b) parallel to each other and disposed symmetrically in relation to the transverse mid-plane (A).

7. The lumbar support belt (1) according to one of claims 1 to 6, **characterized in that** the elastic textile strip has a central portion (3) which receives the posterior boning elements (5a, 5b) arranged in a V-shape, extended by two lateral portions (4a, 4b) which each receive an anterolateral bone (7a, 7b) and an anterior bone (8a, 8b), the central part (3) capable of bearing against the lumbar region of the patient being enlarged in relation to each of the lateral portions (4a, 4b).

8. The lumbar support belt according to one of claims 1 to 7, **characterized in that** the ends of the belt are provided with looped fabric and hooks making it possible to close the belt on itself.

## Patentansprüche

1. Lumbaler Stützgürtel (1), der im unteren Teil des Rumpfes eines Patienten platzierbar ist, einen Streifen (2) aus einem elastischen Textil umfassend, der an jedem seiner Enden mit Befestigungsmitteln des Streifens an sich selbst und mindestens zwei hinteren Versteifungselementen (5a, 5b) versehen ist, die auf der einen und der anderen Seite der transversalen medianen Ebene (A) des Streifens angeordnet sind, wobei der Gürtel auf dem Patienten derart positionierbar ist, dass sich die Enden des Steifens gegenüber dem abdominalen Bereich des Patienten verbinden und sich die hinteren Versteifungselemente (5a, 5b) auf dem Lumbalbereich des Patienten abstützen, wobei die hinteren Versteifungselemente (5a, 5b) derart V-förmig ausgerichtet sind, dass die hinteren Versteifungselemente (5a, 5b) in Richtung der lumbosakralen Region des Patienten konvergieren, **dadurch gekennzeichnet, dass** die hinteren Versteifungselemente aus einem elastischen Material sind.

2. Lumbaler Stützgürtel (1) nach Anspruch 1, **dadurch gekennzeichnet, dass** der elastische Textilstreifen (2) mit mindestens zwei Abnähern (9a, 9b) versehen ist, die im Verhältnis zur Medianebene transversal und symmetrisch sind.

3. Lumbaler Stützgürtel (1) nach Anspruch 1 oder Anspruch 2, **dadurch gekennzeichnet, dass** jedes hintere Versteifungselement (5a, 5b) zwei parallele nebeneinander liegende Versteifungen (6a, 6b) aufweist.

4. Lumbaler Stützgürtel (1) nach einem der Ansprüche 1 bis 3, **dadurch gekennzeichnet, dass** er zwei anterolaterale Versteifungen (7a, 7b) aufweist, die zueinander parallel und im Verhältnis zur transversalen medianen Ebene (A) symmetrisch angeordnet sind.

5. Lumbaler Stützgürtel (1) nach Anspruch 4, **dadurch gekennzeichnet, dass** er einen Abnäher (10a, 10b) etwa über jeder anterolateralen Versteifung (7a, 7b) aufweist.

6. Lumbaler Stützgürtel (1) nach einem der Ansprüche 1 bis 4, **dadurch gekennzeichnet, dass** er zwei vordere Versteifungen (8a, 8b) aufweist, die zueinander parallel und im Verhältnis zur transversalen medianen Ebene (A) symmetrisch angeordnet sind.

7. Lumbaler Stützgürtel (1) nach einem der Ansprüche 1 bis 6, **dadurch gekennzeichnet, dass** der elastische Textilstreifen einen mittleren Abschnitt (3) aufweist, der die hinteren, V-förmig angeordneten Versteifungselemente (5a, 5b) aufnimmt, die von zwei seitlichen Abschnitten (4a, 4b) verlängert werden, von denen jeder eine anterolaterale Versteifung (7a, 7b) und eine vordere Versteifung (8a, 8b) aufnimmt, wobei sich der mittlere Abschnitt (3), der imstande ist, sich auf dem Lumbalbereich des Patienten abzustützen, im Verhältnis zu jedem der seitlichen Abschnitte (4a, 4b) verbreitert.

8. Lumbaler Stützgürtel (1) nach einem der Ansprüche 1 bis 7, **dadurch gekennzeichnet, dass** die Enden des Gürtels mit Stoff mit Schlaufen und Haken versehen sind, die es erlauben, den Gürtel an sich selbst zu schließen.
